# EUROPEAN PATENT SPECIFICATION

(11) **EP 0 383 599 B1**
(45) Date of publication and mention of the grant of the patent: **07.02.1996**
(21) Application number: 90301639.2
(22) Date of filing: 15.02.1990
(51) Int. Cl.: C12N 15/62, C12N 15/31, C12P 21/02, A61K 47/48, A61K 38/00

(54) **Protein anti-cancer agent**
Protein-Antikrebsmittel
Agent anticancéreux protéique

(30) Priority: 17.02.1989 US 312540; 03.08.1989 US 389092; 21.12.1989 US 449187
(43) Date of publication of application: 22.08.1990
(73) Proprietor: MERCK & CO. INC., Rahway New Jersey 07065-0900 (US)
(72) Inventor: Oliff, Allen, Gwynedd Valley, PA 19401 (US); Jones, Deborah D., Lansdale, PA 19446 (US); Edwards, Gwynneth M., Collegeville, PA 19426 (US)
(74) Representative: Jones, Helen Marjorie Meredith

(56) References cited:
- EP-A- 0 192 811
- EP-A- 0 234 599
- EP-A- 0 261 671
- US-A- 4 545 985
- PROCEEDINGS OF THE NATIONAL ACADEMY OF SCIENCES OF USA, vol. 84, no. 13, July 87, pp. 4538-4542, Washington, US; V.K. CHAUDHARY et al.
- BIOTECHNOLOGY, November 1988, pages 1326- 1329, New York, US; BAILON et al.
- EMBASE NO. 88183894 & J. BIOL. CHEM. vol. 264, no. 24, 25th August 1989, pages 14256-14261, Bethesda, US; F. AMANO et al.
- JOURNAL OF BIOLOGICAL CHEMISTRY, vol. 264, no. 26, 15th September 1989, pages 15157-15160, Baltimore, US; I. PASTAN et al.

## Description

### BACKGROUND OF THE INVENTION

Traditional cancer chemotherapy relies on the ability of drugs to kill tumor cells in cancer patients. Unfortunately, these same drugs frequently kill normal cells as well as the tumor cells. The extent to which a cancer drug kills tumor cells rather than normal cells is an indication of the compound's degree of selectivity for tumor cells. One method of increasing the tumor cell selectivity of cancer drugs is to deliver drugs preferentially to the tumor cells while avoiding normal cell populations. Another term for the selective delivery of chemotherapeutic agents to specific cell populations is "targeting". Drug targeting to tumor cells can be accomplished in several ways. One method relies on the presence of specific receptor molecules found on the surface of tumor cells. Other molecules, referred to as "targeting agents", can recognize and bind to these cell surface receptors. These "targeting agents" include, e.g., antibodies, growth factors, or hormones. "Targeting agents" which recognize and bind to specific cell surface receptors are said to target the cells which possess those receptors. For example, many tumor cells possess a protein on their surfaces called the epidermal growth factor receptor. Several growth factors including epidermal growth factor (EGF) and transforming growth factor-alpha (TGF-alpha) recognize and bind to the EGF receptor on tumor cells. EGF and TGF-alpha are therefore "targeting agents" for these tumor cells.

"Targeting agents" by themselves do not kill tumor cells. Other molecules including cellular poisons or toxins can be linked to "targeting agents" to create hybrid molecules that possess both tumor cell targeting and cellular toxin domains. These hybrid molecules function as tumor cell selective poisons by virtue of their abilities to target tumor cells and then kill those cells via their toxin component. Some of the most potent cellular poisons used in constructing these hybrid molecules are bacterial toxins that inhibit protein synthesis in mammalian cells. Pseudomonas exotoxin A is one of these bacterial toxins, and has been used to construct hybrid "targeting - toxin" molecules (U.S. Patent 4,545,985).

Pseudomonas exotoxin A intoxicates mammalian cells by first binding to the cell's surface, then entering the cell cytoplasm and inactivating elongation factor 2 which is a cellular protein required for protein synthesis. Pseudomonas exotoxin A has been used to construct anticancer hybrid molecules using monoclonal antibodies and protein hormones. However, one problem with these hybrid molecules is that they exhibit toxicity towards normal cells. At least part of the toxicity associated with hybrid molecules containing pseudomonas exotoxin A is due to the ability of pseudomonas exotoxin A by itself to bind to and enter many types of mammalian cells. Therefore, hybrid molecules formed between pseudomonas exotoxin A and specific "targeting agents" can bind to many normal cells in addition to the cells recognized by the "targeting agent". One method of dealing with this problem is to modify pseudomonas exotoxin A so that it is no longer capable of binding to normal cells. This can be accomplished by removing that portion of the pseudomonas exotoxin A molecule which is responsible for its cellular binding activity. A truncated form of the pseudomonas exotoxin A molecule has been prepared which retains the ability to inactivate elongation factor 2 but no longer is capable of binding to mammalian cells. This modified pseudomonas exotoxin A molecule is called pseudomonas exotoxin - 40 or PE₄₀ (Hwang et al., Cell 48:129-136 1987).

PE₄₀ has been linked to several targeting molecules including TGF-alpha (Chaudhary et al., PNAS USA 84:4583-4542 1987). In the case of TGF-alpha, hybrid molecules containing PE₄₀ and TGF-alpha domains are capable of specifically binding to tumor cells that possess EGF receptors and intoxicating these cells via inhibiting protein synthesis. In order for this hybrid molecule to efficiently bind to the EGF receptor it must assume the proper conformation. Efficient receptor binding is also dependent or having the "targeting domain" properly exposed so that it is accessible for binding. When TGF-alpha and PE₄₀ hybrid molecules are produced as fusion proteins in bacteria using recombinant DNA techniques the majority of hybrid molecules exhibit poor EGF receptor binding activity.

### DISCLOSURE STATEMENT

1. U.S. patent 4,545,985 teaches that pseudomonas exotoxin A can be conjugated to antibodies or to epidermal growth factor. Patent 4,545,985 further teaches that these conjugates can be used to kill human tumor cells.
2. U.S. patent 4,664,911 teaches that antibodies can be conjugated to the A chain or the B chain of ricin which is a toxin obtained from plants. Patent 4,664,911 further teaches that these conjugates can be used to kill human tumor cells.
3. U.S. patent 4,675,382 teaches that hormones such as melanocyte stimulating hormone (MSH) can be linked to a portion of the diphtheria toxin protein via peptide bonds. Patent 4,675,382 further teaches that the genes which encode these proteins can be joined together to direct the synthesis of a hybrid fusion protein using recombinant DNA techniques. This fusion protein has the ability to bind to cells that possess MSH receptors.
4. Murphy et al., PNAS USA 83:8258-8262 1986, Genetic construction, expression, and melanoma-selective cytotoxicity of a diphtheria toxin-related alpha-melanocyte-stimulating hormone fusion protein. This article teaches that a hybrid fusion protein produced in bacteria using recombinant DNA technology and consisting of a portion of the diphtheria toxin protein joined to alpha-melanocyte-stimulating hormone will bind to and kill human melanoma cells.
5. Kelley et al., PNAS USA 85:3980-3984 1988, Interleukin 2-diphtheria toxin fusion protein can abolish cell-mediated immunity in vivo. This article teaches that a hybrid fusion protein produced in bacteria using recombinant DNA technology and consisting of a portion of the diphtheria toxin protein joined to interleukin 2 functions in nude mice to suppress cell mediated immunity.
6. Allured et al., PNAS USA 83:1320-1324 1986, Structure of exotoxin A of Pseudomonas aeruginosa at 3.0 Angstrom. This article teaches the three dimensional structure of the pseudomonas exotoxin A protein.
7. Hwang et al., Cell 48:129-136 1987, Functional Domains of Pseudomonas Exotoxin Identified by Deletion Analysis of the Gene Expressed in E. Coli. This article teaches that the pseudomonas exotoxin A protein can be divided into three distinct functional domains responsible for: binding to mammalian cells, translocating the toxin protein across lysosomal membranes, and ADP ribosylating elongation factor 2 inside mammalian cells. This article further teaches that these functional domains correspond to distinct regions of the pseudomonas exotoxin A protein.
8. European patent application 0 261 671 published 30 March 1988 teaches that a portion of the pseudomonas exotoxin A protein can be produced which lacks the cellular binding function of the whole pseudomonas exotoxin A protein but possess the translocating and ADP ribosylating functions of the whole pseudomonas exotoxin A protein. The portion of the pseudomonas exotoxin A protein that retains the translocating and ADP ribosylating functions of the whole pseudomonas exotoxin A protein is called pseudomonas exotoxin - 40 or PE-40. PE-40 consists of amino acid residues 252-613 of the whole pseudomonas exotoxin A protein as defined in Gray et al., PNAS USA 81:2645-2649 1984. This patent application further teaches that PE-40 can be linked to transforming growth factor-alpha to form a hybrid fusion protein produced in bacteria using recombinant DNA techniques.
9. Chaudhary et al., PNAS USA 84:4538-4542 1987, Activity of a recombinant fusion protein between transforming growth factor type alpha and Pseudomonas toxin. This article teaches that hybrid fusion proteins formed between PE-40 and transforming growth factor-alpha and produced in bacteria using recombinant DNA techniques will bind to and kill human tumor cells possessing epidermal growth factor receptors.
10. Bailon, Biotechnology, pp. 1326-1329 Nov. 1988. Purification and Partial Characterization of an Interleukin 2-Pseudomonas Exotoxin Fusion Protein. This article teaches that hybrid fusion proteins formed between PE-40 and interleukin 2 and produced in bacteria using recombinant DNA techniques will bind to and kill human cell lines possessing interleukin 2 receptors.

### OBJECTS OF THE INVENTION

It is an object of the present invention to provide modifications of PE₄₀ which permit efficient binding of hybrid molecules formed between "targeting agents" and modified PE₄₀ molecules to growth factor receptors that recognize the "targeting agent". It is another object of this invention to provide a method for recovering the hybrid proteins produced between "targeting agents" and modified PE₄₀ as fusion proteins in bacteria. Another object of the present invention is to provide a hybrid (or fusion) protein having a cell receptor binding domain (or region) and a PE₄₀ domain (or region) wherein the PE₄₀ domain has been modified to improve binding of the hybrid protein to the epidermal growth factor receptor or to the receptor bound by the targeting agent linked to the modified PE₄₀. Another object is to provide a hybrid protein that is more readily purified. These and other objects of the present invention will be apparent from the following description.

### SUMMARY OF INVENTION

The present invention provides a hybrid molecule comprising a modified PE₄₀ domain bonded to a protein targeting domain that binds to a growth factor receptor. The modified PE₄₀ domain improves the receptor binding activity of this hybrid molecule. Substitution of other amino acids such as, e.g., alanine for the cysteine residues in PE₄₀, or deletion of cysteine residues, improves binding of the hybrid molecule to the receptors recognized by the targeting domain. The hybrid molecules of the present invention bind more efficiently to targeted receptors on human tumor cells than hybrid molecules having unmodified PE₄₀.

### DETAILED DESCRIPTION OF THE INVENTION

Hybrid molecules formed between TGF-alpha and PE₄₀ are characterized in three primary assay systems. These assays include: 1 - ADP ribosylation of elongation factor 2 which measures the enzymatic activity of TGF-alpha - PE₄₀ that inhibits mammalian cell protein synthesis, 2 - inhibition of radiolabeled EGF binding to the EGF receptor on membrane vesicles from A431 cells which measures the EGF receptor binding activity of TGF-alpha - PE₄₀, and 3 - cell proliferation as assessed by conversion of 3-[4,5-dimethylthiazol-2-yl]-2,5-diphenyltetrazolium bromide (MTT) to formazan which is used to measure the survival of tumor cells following exposure to TGF-alpha - PE₄₀. These assays are performed as previously described (Dominic et al., Infection and Immunity 16:832-841 1977, Cohen et al., J. Biol. Chem. 257:1523-1531 1982, Riemen et al., Peptides 8:877-885 1987, Mosmann J. Immunol. Methods 65:55-63 1983).

To create new TGF-alpha - PE₄₀ hybrid molecules with superior receptor binding characteristics we first produced a series of recombinant DNA molecules that encoded either TGF-alpha - PE₄₀ or specifically modified versions of TGF-alpha - PE₄₀. The original or parental TGF-alpha - PE₄₀ gene was molecularly cloned in a bacterial TAC expression plasmid vector (pTAC TGF57-PE40) using distinct segments of cloned DNA as described in Example 2. The pTAC TGF57-PE40 DNA clone was used as the starting reagent for constructing specifically modified versions of TGF-alpha - PE₄₀ DNA. The specific modifications of the pTAC TGF57-PE40 DNA involve site specific mutations in the DNA coding sequence required to replace two or four of the cysteine codons within the PE₄₀ domain of the pTAC TGF57-PE40 DNA with codons for other amino acids. Alternatively, the site specific mutations can be engineered to delete two or four of the cysteine codons within the PE40 domain of pTAC TGF57-PE40. The site specific mutations in the pTAC TGF57-PE40 DNA were constructed using the methods of Winter et al., Nature 299:756-758 1982. Specific examples of the mutated pTAC TGF57-PE40 DNAs are presented in Example 3. The amino acid sequence of the hybrid protein encoded by the pTAC TFG57-PE40 DNA is presented in Figure 3. The four cysteine residues in the PE₄₀ domain of the parental TGF-alpha - PE₄₀ hybrid protein are designated residues Cys⁶⁵, Cys⁸⁷, Cys³⁷, and Cys³⁷⁹ (Figure 3). Amino acid residues are numbered as defined in Gray et al, PNAS USA 81: 2645-2649 (1984). The modified TGF-alpha - PE₄₀ hybrid proteins generated from the specifically mutated pTAC TGF57-PE40 DNA contain substitutions or deletions of residues [Cys⁶⁵ and Cys⁸⁷] or [Cys³⁷ and Cys³⁷⁹], or [Cys⁶⁵, Cys⁸⁷, Cys³⁷, and Cys³⁷⁹]. To simplify the nomenclature for describing the modified hybrid proteins produced from these mutated pTAC TGF57-PE40 DNAs we have designated the amino acid residues at positions 265 and 287 the "A" locus and the residues at positions 372 and 379 the "B" locus. When cysteines are present at amino acid residues 265 and 287 as in parental TGF-alpha - PE₄₀ hybrid molecule, the locus is capitalized (i.e. "A"). When the cysteines are substituted with other amino acids such as, for example, alanine, phenylalanine, valine, leucine or isoleucine, or deleted from residues 265 and 287 the locus is represented by a lower case "a". Similarly, if the amino acid residue at positions 372 and 379 are cysteines the locus is represented by an upper case "B" while a lower case "b" represents this locus when the amino acid residues at positions 372 and 379 are substituted with other amino acids or deleted. Thus when all four cysteine residues in the PE₄₀ domain of TGF-alpha - PE₄₀ are substituted with alanines the modified hybrid protein is designated TGF-alpha - PE₄₀ ab. In a similar fashion the parental TGF-alpha - PE₄₀ hybrid protein with cysteines at amino acid residue positions 265, 287, 372 and 379 can be designated TGF-alpha - PE₄₀ AB.

Both the TGF-alpha - PE₄₀ AB hybrid protein and the modified TGF-alpha - PE₄₀ hybrid proteins are produced in E. coli using the TAC expression vector system described by Linemeyer et al., Bio Technology 5:960-965 1987. The recombinant hybrid proteins produced in these bacteria are harvested and purified by lysing the bacteria in guanidine hydrochloride followed by the addition of sodium sulphite and sodium tetrathionate. This reaction mixture is subsequently dialyzed and urea is added to solubilize proteins that have precipitated out of solution. The mixture is next centrifuged to remove insoluble proteins and the recombinant hybrid TGF-alpha - PE₄₀ proteins are separated using ion exchange chromatography followed by size exclusion chromatography, followed once again by ion exchange chromatography. The purified TGF-alpha - PE₄₀ hybrid proteins are next exposed to reducing agents such as beta-mercaptoethanol in' order to permit disulfide bonds to form within the hybrid protein between pairs of cysteine residues. Finally, the refolded hybrid proteins are subjected to size exclusion and ion exchange chromatography to isolate highly pure TGF-alpha - PE₄₀ protein. The precise details of this purification scheme are described in Example 2. Once purified and refolded the biologic activity of these hybrid proteins can be characterized using the ADP ribosylation, EGF receptor binding, and cell proliferation assays described above.

An important utility of TGF-alpha -PE₄₀ lies in its ability to bind to and kill cells possessing EGF receptors. Many human tumor cells possess EGF receptors and therefore are susceptible to the cell-killing effects of TGF-alpha - PE₄₀. Other non-cancerous human cells including keratinocytes possess EGF receptors and are also susceptible to the cell-killing activity of TGF-alpha - PE₄₀. Several human diseases are characterized by increased proliferation of keratinocytes including psoriasis and warts.

The following examples illustrate the present invention without, however, limiting the same thereto. All of the enzymatic reactions required for molecular biology manipulations, unless otherwise specified, were carried out as described in Maniatis et al. (1982) In: Molecular Cloning: A Laboratory Manual, Cold Spring Harbor Press.

### EXAMPLE 1

### Production and isolation of recombinant TGF-alpha - PE₄₀ fusion proteins:

### Production of fusion protein

Transformed E. coli JM-109 cells were cultured in 1L shake flasks in 500 ml LB-Broth in the presence of 100 µg/ml ampicillin at 37°C. After the A600 spectrophotometric absorbance value reached 0.6, isopropyl B-D-thio-galactopyranoside was added to a final concentration of 1 mM. After 2 hours the cells were harvested by centrifugation.

### S-Sulphonation of fusion protein

The cells were lysed in 8M guanidine hydrochloride, 50 mM Tris pH 8.0, 1 mM EDTA by stirring at room temperature for 2 hours. The lysis mixture was brought to 0.4 M sodium sulphite and 0.1M sodium tetrathionate by adding solid reagents and the pH was adjusted to 9.0 with 1M NaOH. The reaction was allowed to proceed at room temperature for 16 hours.

### Preparation for chromatography

The protein solution was dialysed against a 10,000 fold excess volume of 1mM EDTA at 4°C. The mixture was then brought to 6M urea, 50 mM Tris pH 8.0, 50 mM NaCl at room temperature and stirred for 2 hours. Any undissolved material was removed by centrifugation at 32,000 x g for 30 minutes.

### DEAE F.F. Sepharose Chromatography

The cleared supernatant from the previous step was applied to a 26 x 40 cm DEAE Fast Flow column (Pharmacia LKB Biotechnology Inc.) equilibrated with 6M urea, 50 mM Tris pH 8.0, 50 mM NaCl at a flow rate of 1 ml/minute. The column was washed with the equilibration buffer until all unadsorbed materials were removed as evidenced by a UV 280 spectrophotometric absorbance below 0.1 in the equilibration buffer as it exits the column. The adsorbed fusion protein was eluted from the column with a 1000 ml 50-350 mM NaCl gradient and then concentrated in a stirred cell Amicon concentrator fitted with a YM-30 membrane.

### Sephacryl S-300

The concentrated fusion protein (8 mls) was applied to a 2.6 x 100 cm Sephacryl S-300 column (Pharmacia LKB Biotechnology Inc.) equilibrated with 6M urea, 50 mM Tris pH 8.0, 50 mM NaCl at a flow rate of 0.25 ml/minute. The column was eluted with additional equilibration buffer and 3 ml fractions collected. Fractions containing TGF-alpha - PE₄₀ activity were pooled.

### Q-sepharose Chromatography

The pooled fractions from the S-300 column were applied to a 1.6 x 40 cm Q-sepharose column (Pharmacia LKB Biotechnology, Inc.) equilibrated with 6M urea, 50 mM Tris pH 8.0, 50 mM NaCl at a flow rate of 0.7 ml/minute. The column was washed with the equilibration buffer and then eluted with a 600 ml 50-450 mM NaCl gradient. The fractions containing the TGF-alpha - PE₄₀ activity were pooled and then dialysed against 50 mM glycine pH 9.0 and stored at -20°C.

### Refolding

A sample of the protein was thawed and diluted to a spectrophotometric absorbance at UV A280 = 0.1 in 50 mM glycine pH 10.5. Beta-mercaptoethanol was added to give a 4:1 molar ratio over the theoretical number of S-sulphonate groups present in the protein sample. The reaction was allowed to proceed for 16 hours at 4°C after which time the solution was dialysed against a 10,000 fold excess of physiologically buffered saline and stored at -20°C.

### Example 2

### Construction of recombinant DNA clones containing TGF-alpha - PE₄₀ DNA

The TGF-alpha DNA segment was constructed using three sets of synthetic oligonucleotides as described by Defeo-Jones et al., Molecular and Cellular Biology 8:2999-3007 1988. This synthetic TGF-alpha gene was cloned into pUC-19. DNA from the pUC-19 clone containing recombinant human TGF-alpha was digested with Sph I and Eco RI. The digestion generated a 2.8 kb DNA fragment containing all of pUC-19 and the 5' portion of TGF-alpha. The 2.8 kb fragment was purified and isolated by gel electrophoresis. An Eco RI to Sph I oligonucleotide cassette was synthesized. This synthetic cassette had the sequence indicated below:

5'-CGGACCTCCTGGCTGCGCATCTAGG-3'
3'-GTACGCCTGGAGGACCGACGCGTAGATCCTTAA-5'

For convenience, this oligonucleotide cassette was named 57. Cassette 57 was annealed and ligated to the TGF-alpha containing 2.8 kb fragment forming a circularized plasmid. Clones which contained the cassette were identified by hybridization to radiolabeled cassette 57 DNA. The presence of human TGF-alpha was confirmed by DNA sequencing. Sequencing also confirmed the presence of a newly introduced Fsp I site at the 3' end of the TGF-alpha sequence. This plasmid, named TGF-alpha-57/pUC-19, was digested with HinD III and Fsp I which generated a 168 bp fragment containing the TGF-alpha gene (TGF-alpha-57). A separate preparation of pUC-19 was digested with HinD III and Eco RI which generated a 2.68 kb pUC-19 vector DNA. The PE₄₀ DNA was isolated from plasmid pVC 8 (Chaudhary et al., PNAS USA 84:4538-4542 1987). pVC 8 was digested using Nde I. A flush end was then generated on this DNA by using the standard conditions of the Klenow reaction (Maniatis, et al., supra, p.113). The flush-ended DNA was then subjected to a second digestion with Eco RI to generate a 1.3 kb Eco RI to Nde I (flush ended) fragment containing PE₄₀. The TGF-alpha-57 HinD III to Fsp I fragment (168 bp) was ligated to the 2.68 kb pUC-19 vector. Following overnight incubation, the 1.3 kb EcoRI to Nde I (flush ended) PE₄₀ DNA fragment was added to the ligation mixture. This second ligation was allowed to proceed overnight. The ligation reaction product was then used to transform JM 109 cells. Clones containing TGF-alpha-57 PE₄₀ in pUC-19 were identified by hybridization to radiolabeled TGF-alpha-57 PE₄₀ DNA and the DNA from this clone was isolated. The TGF-alpha-57 PE₄₀ was removed from the pUC-19 vector and transferred to a TAC vector system described by Linemeyer et al., Bio-Technology 5:960-965 1987). The TGF-alpha-57 PE₄₀ in pUC-19 was digested with HinD III and Eco RI to generate a 1.5 kb fragment containing TGF-alpha-57 PE₄₀. A flush end was generated on this DNA fragment using standard Klenow reaction conditions (Maniatis et al., loc. cit.). The TAC vector was digested with HinD III and Eco RI. A flush end was generated on the digested TAC vector DNA using standard Klenow reaction conditions (Maniatis et al., loc. cit. The 2.7 kb flush ended vector was isolated using gel electrophoresis. The flush ended TGF-alpha-57 PE₄₀ fragment was then ligated to the flush ended TAC vector. The plasmid generated by this ligation was used to transform JM 109 cells. Candidate clones containing TGF-alpha-57 PE₄₀ were identified by hybridization as indicated above and sequenced. The clone containing the desired construction was named pTAC TGF57-PE40. The plasmid generated by these manipulations is depicted in Table 1. The nucleotide sequence of the amino acid codons of the TGF-alpha - PE₄₀ fusion protein encoded in the pTAC TGF-57-PE40 DNA are depicted in Table 2. The amino acid sequence encoded by the TGF-57-PE40 gene is shown in Table 3.

### Example 3

### Construction of modified versions of recombinant TGF-alpha - PE₄₀ containing DNA clones: Substitution of alanines for cysteines.

### TGF-alpha - PE₄₀ aB:

The clone pTAC TGF57-PE40 was digested with SphI and BamHI and the 750 bp SphI-BamHI fragment (specifying the C-terminal 5 amino acids of TGF-alpha and the N-terminal 243 amino acids of PE₄₀) was isolated. M13 mp19 vector DNA was cut with SphI and BamHI and the vector DNA was isolated. The 750 bp SphI-BamHI TGF-alpha - PE₄₀ fragment was ligated into the M13 vector DNA overnight at 15°C. Bacterial host cells were transformed with this ligation mixture, candidate clones were isolated and their plasmid DNA was sequenced to insure that these clones contained the proper recombinant DNAs. Single stranded DNA was prepared for mutagenesis.

An oligonucleotide (oligo #132) was synthesized and used in site directed mutagenesis to introduce a HpaI site into the TGF-alpha - PE₄₀ DNA at amino acid position 272 of PE₄₀:

5' CTGGAGACGTTAACCCGTC 3' (oligo #132)

One consequence of this site directed mutagenesis was the conversion of residue number 272 in PE₄₀ from phenylalanine to leucine. The mutagenesis was performed as described by Winter et al., Nature, 299:756-758 1982.

A candidate clone containing the newly created HpaI site was isolated and sequenced to validate the presence of the mutated genetic sequence. This clone was then cut with SphI and SalI. A 210 bp fragment specifying the C-terminal 5 amino acids of TGF-alpha and the N-terminal 70 amino acids of PE₄₀ and containing the newly introduced HpaI site was isolated and subcloned back into the parent pTAC TGF57-PE40 plasmid at the SphI-SalI sites. Bacterial host cells were transformed, a candidate clone was isolated and its plasmid DNA was sequenced to insure that this clone contained the proper recombinant DNA. For convenience this clone was named pTAC TGF57-PE40-132. pTAC TGF57-PE40-132 was digested with SphI and HpaI and a 3.96 Kb DNA fragment was isolated. A synthetic oligonucleotide cassette (oligo #153) spanning the C-terminal 5 amino acids of TGF-alpha and the N-terminal 32 amino acids of PE₄₀ and containing SphI and HpaI compatible ends was synthesized and ligated to the digested pTAC TGF57-PE40-132:

5'     CGGACCTCCTGGCCATGGCCGAAGAGGGCGGCAGCCTGGCCGCGCTGACCGCGCA
3' GTACGCCTGGAGGACCGGTACCGGCTTCTCCCGCCGTCGGACCGGCGCGACTGGCGCGT

CCAGGCTGCACACCTGCCGCTGGAGACGTT 3'
GGTCCGACGTGTGGACGGCGACCTCTGCAA 5'    (oligo #153)

This oligonucleotide cassette incorporated a change in the TGF-alpha - PE₄₀ DNA so that the codon specifying cysteine at residue 265 now specified alanine. For convenience this plasmid DNA was called pTAC TGF57-PE40-132,153. Bacterial host cells were transformed with pTAC TGF57-PE40-132,153 DNA. Candidate clones were identified by hybridization, isolated and their plasmid DNA was sequenced to insure that it contained the proper recombinant DNA.

pTAC TGF57-PE40-132,153 DNA was digested with HpaI and SalI and a 3.95 Kb vector DNA was isolated. A synthetic oligonucleotide cassette (oligo #142) spanning amino acid residues 272 to 309 of PE₄₀ and containing HpaI and SalI compatible ends was synthesized and ligated to the 3.95 Kb pTAC TGF/PE40 132,153 DNA.

5' AACCCGTCATCGCCAGCCGCGCGGCTGGGAACAACTGGAGCAGGCTGGCTATCCGGTGC
3' TTGGGCAGTAGCGGTCGGCGCGCCGACCCTTGTTGACCTCGTCCGACCGATAGGCCACG

AGCGGCTGGTCGCCCTCTACCTGGCGGCGCGGCTGTCGTGGAACCAGG 3'
TCGCCGACCAGCGGGAGATGGACCGCCGCGCCGACAGCACCTTGGTCCAGCT 5' (oligo #142)

This oligonucleotide cassette changes the codon specifying cysteine at residue 287 so that this codon now specified alanine. For convenience this mutated plasmid DNA was called pTAC TGF57-PE40-132,153,142. Bacterial host cells were transformed with this plasmid and candidate clones were identified by hybridization. These clones were isolated and their plasmid DNA was sequenced to insure that it contained the proper recombinant DNA. The pTAC TGF57-PE40-132,153,142 plasmid encodes the TGF-alpha - PE₄₀ variant with both cysteines at locus "A" replaced by alanines. Therefore, following the nomenclature described previously this modified version of TGF-alpha - PE₄₀ is called TGF-alpha - PE₄₀ aB. The amino acid sequence encoded by the TGF-alpha-PE₄₀ aB gene is show in Table 4.

### TGF-alpha - PE₄₀ Ab:

The clone pTAC TGF57-PE40 was digested with SphI and BamHI and the 750 bp SphI-BamHI fragment (specifying the C-terminal 5 amino acids of TGF-alpha and the N-terminal 252 amino acids of PE₄₀) was isolated. M13 mp19 vector DNA was cut with SphI and BamHI and the vector DNA was isolated. The 750 bp SphI-BamHI TGF-alpha - PE₄₀ fragment was ligated into the M13 vector DNA overnight at 15°C. Bacterial host cells were transformed with this ligation mixture, candidate clones were isolated and their plasmid DNA was sequenced to insure that these clones contained the proper recombinant DNAs. Single stranded DNA was prepared for mutagenesis.

An oligonucleotide (oligo #133) was synthesized and used in site directed mutagenesis to introduce a BsteII site into the TGF-alpha - PE₄₀ DNA at amino acid position 369 of PE₄₀:

5' GACGTGGTGACCCTGAC 3' (oligo #133)

One consequence of this mutagenesis was the conversion of the serine residue at position 369 of PE₄₀ to a threonine.

A DNA clone containing the newly created BsteII site was identified, isolated and sequenced to ensure the presence of the proper recombinant DNA. This clone was next digested with ApaI and SalI restriction enzymes. A 120 bp insert DNA fragment containing the newly created BsteII site was isolated and ligated into pTAC TGF57-PE40 that had also been digested with ApaI and SalI. Bacterial host cells were transformed, and a candidate clone was isolated and sequenced to insure that the proper recombinant DNA was present. This newly created plasmid DNA was called pTAC TGF57-PE40-133. It was digested with BsteII and ApaI and 2.65 Kb vector DNA fragment was isolated.

A BsteII to ApaI oligonucleotide cassette (oligo #155) was synthesized which spanned the region of TGF-alpha - PE₄₀ deleted from the pTAC TGF57-PE40-133 clone digested with BsteII and ApaI restriction enzymes. This cassette also specified the nucleotide sequence for BsteII and ApaI compatible ends.

5' GTGACCCTGACCGCGCCGGTCGCCGCCGGTGAAGCTGCGGCC 3'
    3' GGACTGGCGCGGCCAGCGGCGGCCACTTCGACGC 5' (oligo #155)

This oligonucleotide cassette changed the codons for cysteines at residues 372 and 379 of PE₄₀ to codons specifying alanines. Oligonucleotide cassette #155 was ligated to the 2.65 Kb vector DNA fragment. Bacterial host cells were transformed and candidate clones were isolated and sequenced to insure that the proper recombinant DNA was present. This newly created DNA clone was called pTAC TGF57-PE40-133,155. It encodes the TGF-alpha - PE₄₀ variant with both cysteines at locus "B: replaced by alanines. Therefore, following the nomenclature described previously this modified version of TGF-alpha - PE₄₀ is called TGF-alpha - PE₄₀ Ab. The amino acid sequence encoded by the TGF-alpha-PE₄₀ Ab gene is shown in Table 5.

### TGF-alpha - PE₄₀ ab:

The pTAC-TGF57-PE40-132,153,142 plasmid encoding TGF-alpha - PE₄₀ aB was digested with SalI and ApaI and the resultant 3.8 Kb vector DNA fragment was isolated. The pTAC TGF57-PE40-133,155 plasmid encoding TGF-alpha - PE₄₀ Ab was also digested with SalI and ApaI and the resultant 140 bp DNA fragment containing the cysteine to alanine changes at amino acid residues 372 and 379 of PE₄₀ was isolated. These two DNAs were ligated together and used to transform bacterial host cells. Candidate clones were identified by hybridization with a radiolabeled 140 bp DNA from pTAC TGF57-PE40-133,155. Plasmid DNA from the candidate clones was isolated and sequenced to insure the presence of the proper recombinant DNA. This newly created DNA clone was called pTAC TGF57-PE40-132,153,142,133,155. This plasmid encodes the TGF-alpha - PE₄₀ variant with all four cysteines at loci "A" and "B" replaced by alanines. Therefore, following the nomenclature described previously this modified version of TGF-alpha - PE₄₀ is called TGF-alpha - PE₄₀ ab. The amino acid sequence encoded by the TGF-alpha-PE₄₀ ab gene is shown in Table 6.

### Example 4

### Construction of modified versions of recombinant TGF-alpha-PE₄₀ containing DNA clones: Selection of cysteine residues

TGF-alpha-PE₄₀ aB, TGF-alpha-PE₄₀ Ab, and TGF-alpha-PE₄₀ ab can also be constructed by removing the cysteine residues at locus "A" and/or locus "B". Construction of these versions of TGF-alpha-PE₄₀ are accomplished identically as described in Example 3 except that: for TGF-alpah-PE₄₀ aB oligonucleotide cassette 153 is changed such that the alanine codon intended for position 265 is deleted and oligonucleotide cassette 142 is changed such that the alanine codon intended for position 287 is deleted. For TGF-alpha-PE₄₀ Ab oligonucleotide cassette 155 is changed such that the alanine codons intended for residues 372 and 379 are deleted. For TGF-alpha-PE₄₀ ab the DNA fragments used to construct this recombinant gene are taken from the TGF-alpha-PE₄₀ aB and TGF-alpha-PE₄₀ Ab gene described in this example.

### Example 5

### Biologic activities of TGF-alpha - PE₄₀ AB, TGF-alpha - PE₄₀ Ab, TGF-alpha - PE₄₀ aB, and TGF-alpha - PE₄₀ ab proteins

The hybrid fusion proteins TGF-alpha - PE₄₀ AB, TGF-alpha - PE₄₀ Ab, TGF-alpha - PE₄₀ aB, TGF-alpha - PE₄₀ ab were expressed in bacterial hosts and isolated as described in Example 1. Each protein was then characterized for its ability to inhibit the binding of radiolabeled epidermal growth factor to the epidermal growth factor receptor on A431 cell membrane vesicles and for its ability to kill A431 cells as measured in MTT cell proliferation assays described previously. The following table summarizes the biologic activites of these proteins:

| | EPIDERMAL GROWTH FACTOR RECEPTOR BINDING | A431 CELL KILLING |
|---|---|---|
| | IC₅₀ nM | EC₅₀ pM |
| TGF-alpha - PE₄₀ AB | 346 | 47 |
| TGF-alpha - PE₄₀ Ab | 588 | 25 |
| TGF-alpha - PE₄₀ aB | 27 | 151 |
| TGF-alpha - PE₄₀ ab | 60 | 392 |

### Example 6

### Substitution of other "targeting agents" that bind to the epidermal growth factor receptor for the TGF-alpha domain of TGF-alpha - PE₄₀ ab

The utility of TGF-alpha - PE₄₀ lies in its ability to bind to and kill cells possessing epidermal growth factor receptors. Other "targeting agents" can be used to create hybrid molecules with the modified PE₄₀ of the present invention that will bind to EGF receptors. For example, the genes for epidermal growth factor or urogastrone or the Shope fibroma virus growth factor, or the vaccinia virus growth factor can be linked to the gene for PE₄₀ and used to direct the synthesis of epidermal growth factor - PE₄₀, or urogastrone - PE₄₀, Or Shope fibroma virus growth factor - PE₄₀, or vaccinia virus growth factor - PE₄₀ hybrid fusion proteins. However, in each case one or more of the modifications to PE₄₀ described herein improves the binding of these other hybrid fusion proteins to cells possessing epidermal growth factor receptors.

### Example 7

### Substitution of other "targeting agents" that bind to other receptors on mammalian cells for the TGF-alpha domain of TGF-alpha - PE₄₀.

It is to be understood that this invention is directed to modification of the PE₄₀ domain of hybrid fusion proteins between PE₄₀ and other "targeting agents" that recognize specific receptors on mammalian cells. For example, fusion proteins formed between proteins and modified PE₄₀ of the present invention of the general formula: protein X - PE₄₀ where protein X is interleukin-2, or interleukin-3, or interleukin-4, or interleukin-6, or platelet derived growth factor, or any other protein that recognizes and binds to a specific mammalian cell receptor have improved binding properties to their respective cellular receptors.

### Example 8

### Bilogic Activity of TGF-alpha - PE₄₀ ab against human keratinocytes

Using the cell proliferation assay of Mossmann, J. Immunol. Methods 65: 55-63 (1983), TGF-alpha - PE₄₀ ab readily killed the human keratinocytes used in the assay. The concentration of TGF-alpha - PE₄₀ required to kill 50% of the keratinocytes (ED₅₀) was 11 nM.

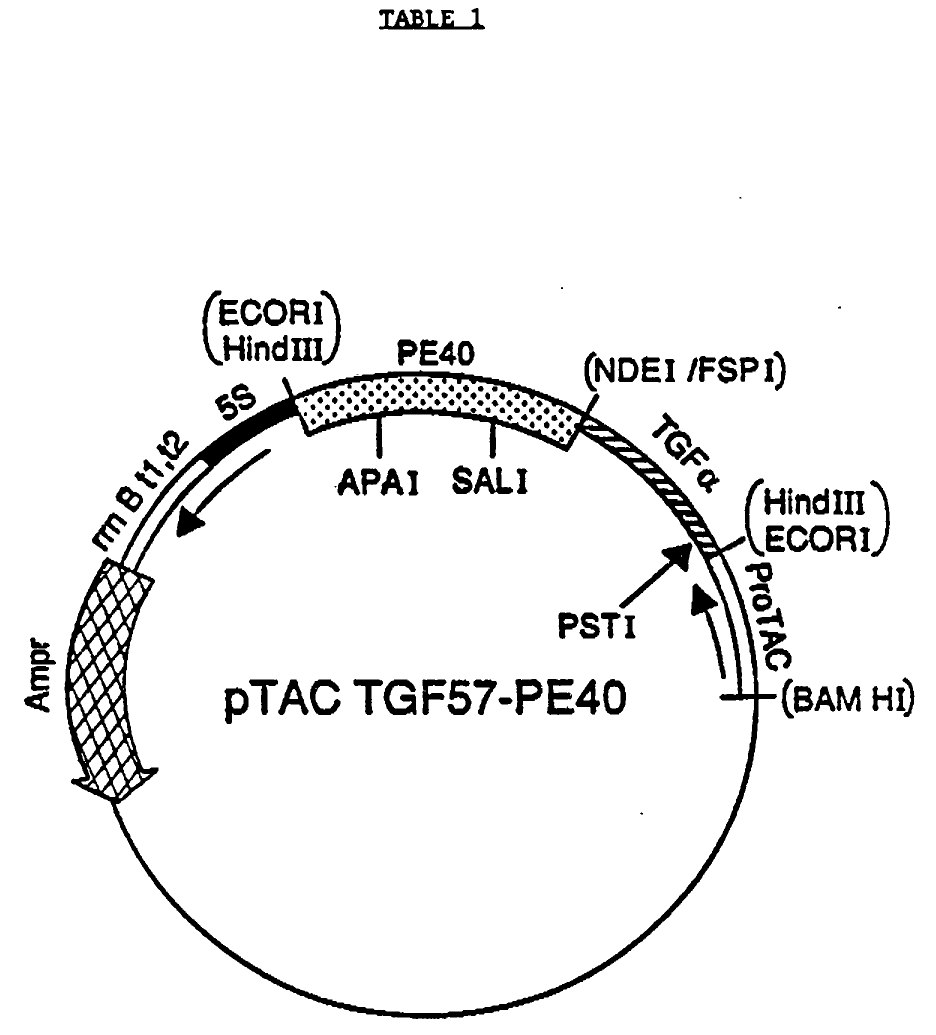

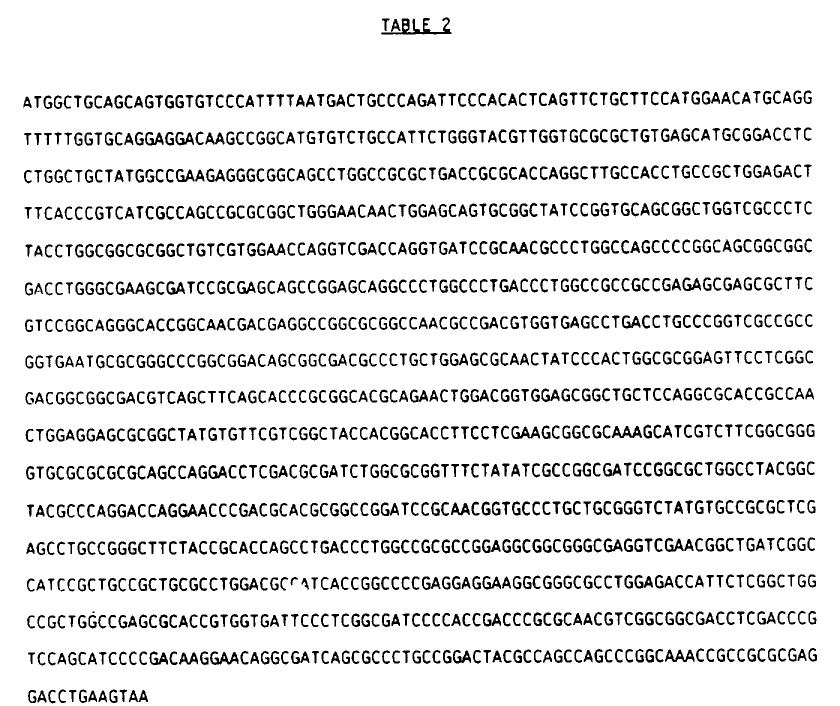

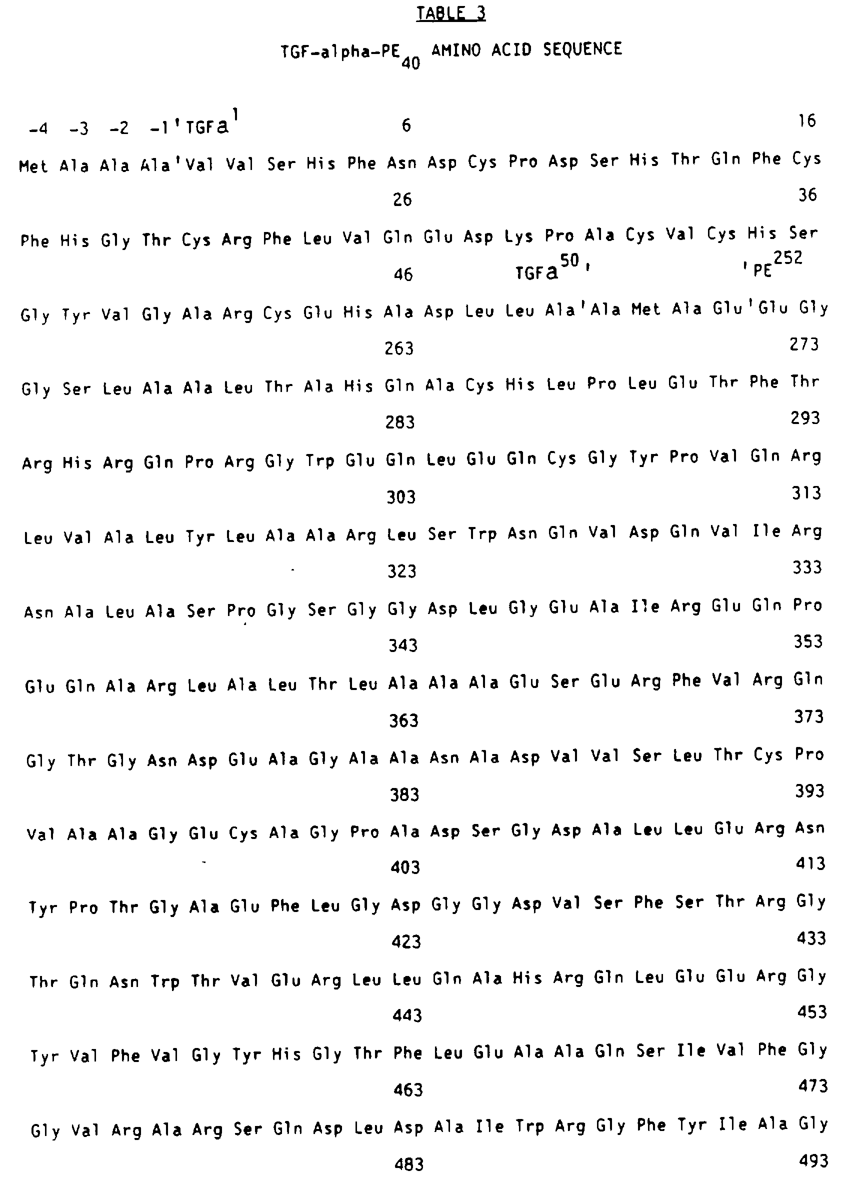

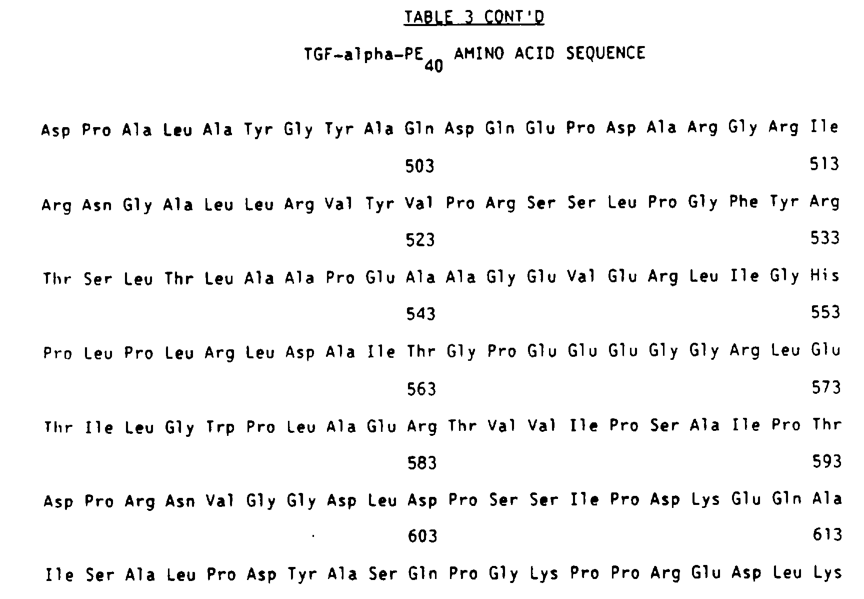

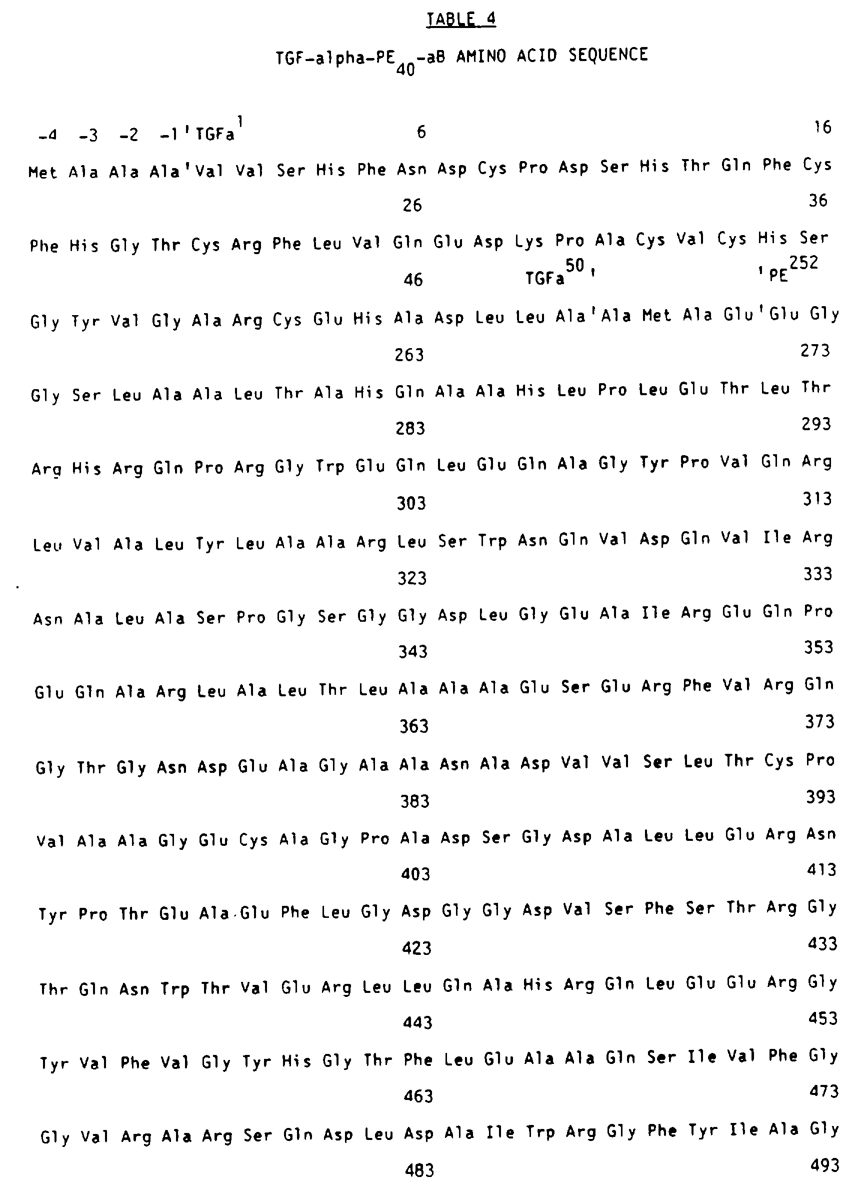

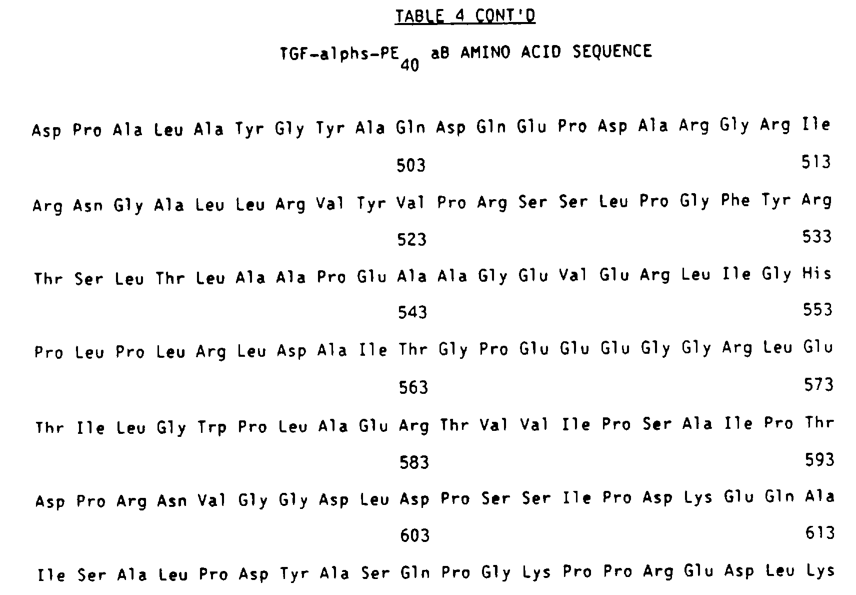

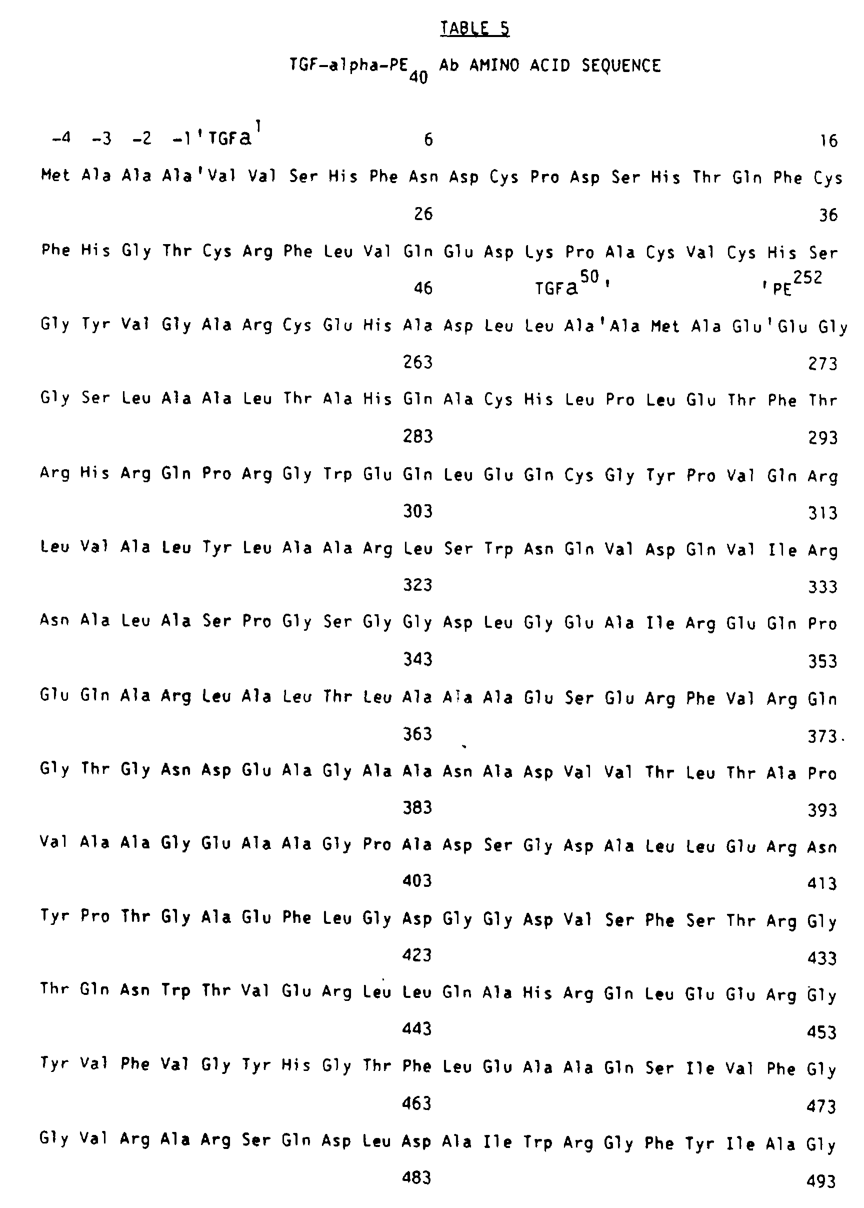

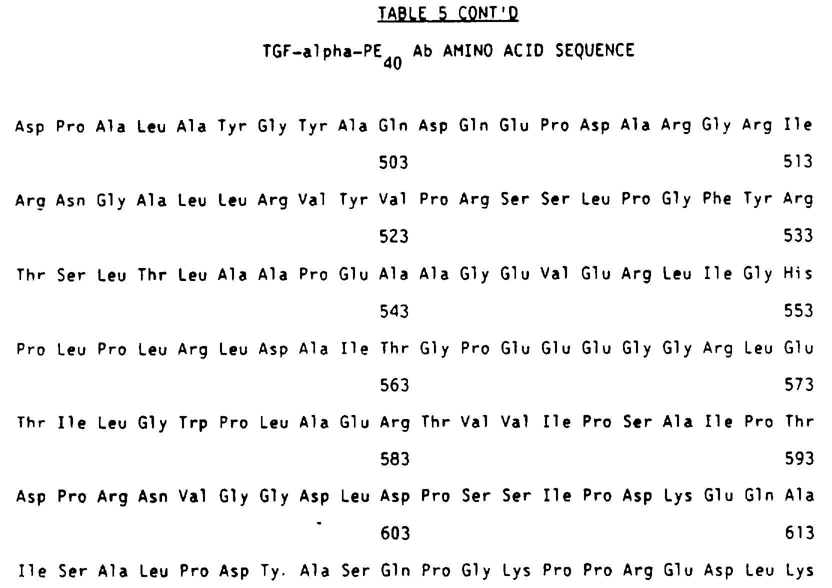

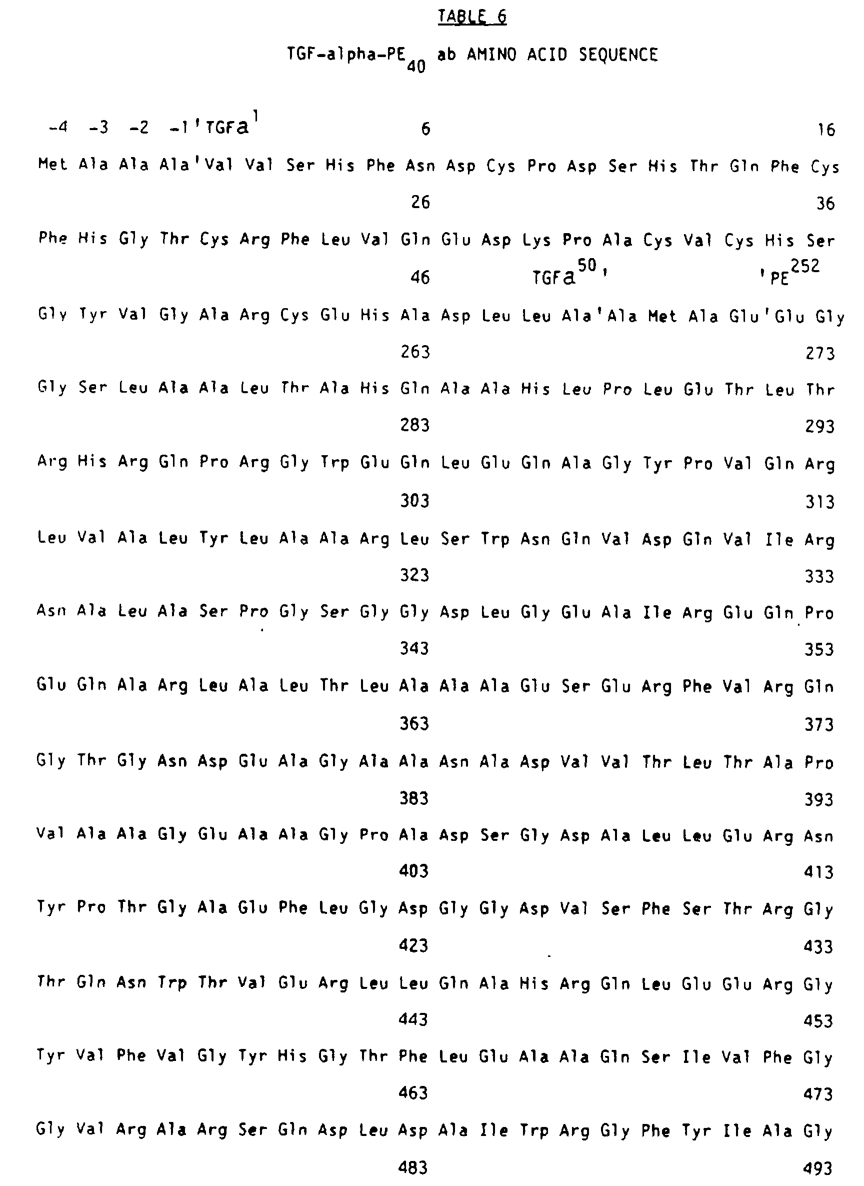

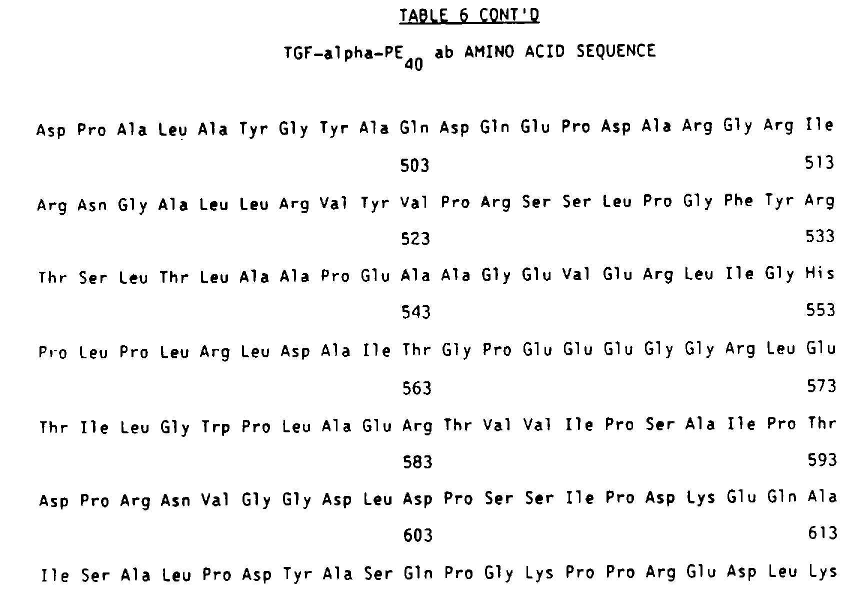

## Claims (Claims for the following Contracting State(s): DK, DE, GB, FR, IT, NL, SE, LI, CH, BE, AT, LU)

1. A hybrid protein comprising a modified PE₄₀ protein, wherein said PE₄₀ protein has been modified to replace at least two of its cysteine residues with the same number of other amino acids that may be the same or different provided they do not form a disulfide bond, and wherein said modified PE₄₀ protein or fragment is bonded to a protein targeting domain that binds to a growth factor receptor.

2. The hybrid protein of claim 1, wherein at least two of said cysteine residues which are modified comprise residues 265 and 287 of the PE₄₀ protein.

3. The hybrid protein of claim 1, wherein at least two of said cysteine residues which are modified comprise residues 372 and 379 of the PE₄₀ protein.

4. The hybrid protein of any of claims 1 to 3, wherein the modified PE₄₀ protein has been modified to replace the cysteine residues at positions 265, 287, 372 and 379.

5. The hybrid protein of any of claims 1 to 4, wherein the cysteine residues are changed to an amino acid selected from the group consisting of alanine, phenylalanine, leucine, isoleucine, and valine.

6. The hybrid protein of claim 5, wherein at least two of the cysteine residues are changed to alanine.

7. The hybrid protein of claim 6, wherein said two cysteine residues are at positions 265 and 287 of the PE₄₀ protein.

8. The hybrid protein of claim 6, wherein said two cysteine residues are at positions 372 and 379 of the PE₄₀ protein.

9. The hybrid protein of any of claims 6 to 8, wherein four cysteine residues are replaced by alanine.

10. The hybrid protein of claim 9, wherein the four cysteine residues are at positions 265, 287, 372 and 379.

11. The hybrid protein of claim 1 or claim 8 which is TGF-alphaPE₄₀ hybrid protein in which the cysteine residues at positions 372 and 379 are modified and in which the cysteine residues at positions 265 and 287 are unmodified.

12. The hybrid protein of claim 1 or claim 10 which is a TGF-alphaPE₄₀ hybrid protein in which the cysteine residues at positions 265, 287, 372 and 379 are modified.

13. The hybrid protein of claim 1 or claim 7 which is a TGF-alphaPE₄₀ hybrid protein in which the cysteine residues at positions 265 and 287 are modified and the cysteine residues at positions 372 and 379 are unmodified.

14. A DNA sequence which encodes for the hybrid protein of any of claims 1 to 13.

15. A plasmid containing the DNA sequence of claim 14 which provides for the expression of the hybrid protein DNA sequence in a recombinant host cell.

16. A method of producing the plasmid of claim 15 to produce said hybrid protein comprising transforming a suitable host cell with said plasmid and culturing said transformed host cell under conditions which provide for expression of the hybrid protein.

17. A composition containing a cytotoxic effective amount of the hybrid protein of any of claims 1 to 13 and a physiologically acceptable carrier.

18. The use of the hybrid protein of any of claims 1 to 13 for the preparation of a composition useful for producing selective cytotoxic activity in a mammalian species.

19. The use as claimed in claim 18 wherein the composition contains a physiologically acceptable carrier.

20. The use of the hybrid protein according to any of claims 1 to 13 for the manufacture of a medicament for reducing the proliferation of keratinocytes.

## Claims (Claims for the following Contracting State(s): ES, GR)

1. A process for producing a hybrid protein comprising a modified PE₄₀ protein, wherein a PE₄₀ protein is modified by replacement at least two cysteine residues by the same number of other amino acids that may be the same or different provided they do not form a disulfide bond, bonded to a protein targeting domain that binds to a growth factor receptor, comprising inserting a plasmid containing DNA encoding said hybrid protein into a suitable procaryotic or eucaryotic host cell and growing the host cell under conditions whereby the hybrid protein is produced.

2. A process according to claim 1, wherein at least two of said cysteine residues which are modified comprise residues 265 and 287 of the PE₄₀ protein.

3. A process according to claim 1, wherein at least two of said cysteine residues which are modified comprise residues 372 and 379 of the PE₄₀ protein.

4. A process according to any of claims 1 to 3, where the modified PE₄₀ protein has been modified to replace the cysteine residues at positions 265, 287, 372 and 379.

5. A process according to any of claims 1 to 4, wherein the cysteine residues are changed to an amino acid selected from the group consisting of alanine, phenylalanine, leucine, isoleucine, and valine.

6. A process according to claim 5, wherein at least two of the cysteine residues are changed to alanine.

7. A process according to claim 6, wherein said two cysteine residues are at positions 265 and 287 of the PE₄₀ protein.

8. A process according to claim 6, wherein said two cysteine residues are at positions 372 and 379 of the PE₄₀ protein.

9. A process according to any of claims 6 to 8, wherein four cysteine residues are replaced by alanine.

10. A process according to claim 9, wherein the four cysteine residues are at positions 265, 287, 372 and 379.

11. A process according to claim 1 or claim 8 wherein the modified protein is a TGF-alphaPE₄₀ hybrid protein in which the cysteine residues at positions 372 and 379 are modified and the cysteine residues at positions 265 and 287 are unmodified.

12. A process according to claim 1 or claim 10 wherein the modified protein is a TGF-alphaPE₄₀ hybrid protein in which the cysteine residues at positions 265, 287, 372 and 379 are modified.

13. A process according to claim 1 or claim 7 wherein the modified protein is a TGF-alphaPE₄₀ hybrid protein in which the cysteine residues at positions 265 and 287 are modified and the cysteine residues at positions 372 and 379 are unmodified.

## Patentansprüche (Patentansprüche für folgende(n) Vertragsstaat(en): DK, DE, GB, FR, IT, NL, SE, LI, CH, BE, AT, LU)

1. Hybridprotein, umfassend ein modifiziertes PE₄₀-Protein, worin das PE₄₀-Protein modifiziert worden ist, um mindestens zwei seiner Cysteinreste durch dieselbe Anzahl anderer Aminosäuren zu ersetzen, welche gleich oder verschieden sein können, mit der Maßgabe, daß sie keine Disulfid-Bindung bilden, und worin das modifizierte PE₄₀-Protein oder Fragment an eine zielerkennende ("targeting") Proteindomäne gebunden ist, welche an einen Wachstumsfaktor-Rezeptor bindet.

2. Hybridprotein nach Anspruch 1, worin mindestens zwei der modifizierten Cysteinreste die Reste 265 und 287 des PE₄₀-Proteins umfassen.

3. Hybridprotein nach Anspruch 1, worin mindestens zwei der modifizierten Cysteinreste die Reste 372 und 379 des PE₄₀-Proteins umfassen.

4. Hybridprotein nach irgendeinem der Ansprüche 1 bis 3, worin das modifizierte PE₄₀-Protein modifiziert worden ist, um die Cysteinreste an den Positionen 265, 287, 372 und 379 zu ersetzen.

5. Hybridprotein nach irgendeinem der Ansprüche 1 bis 4, worin die Cysteinreste gegen eine Aminosäure ausgetauscht sind, die aus der Gruppe aus Alanin, Phenylalanin, Leucin, Isoleucin und Valin ausgewählt ist.

6. Hybridprotein nach Anspruch 5, worin mindestens zwei der Cysteinreste gegen Alanin ausgetauscht sind.

7. Hybridprotein nach Anspruch 6, worin die beiden Cysteinreste an den Positionen 265 und 287 des PE₄₀-Proteins sind.

8. Hybridprotein nach Anspruch 6, worin die beiden Cysteinreste an den Positionen 372 und 379 des PE₄₀-Proteins sind.

9. Hybridprotein nach irgendeinem der Ansprüche 6 bis 8, worin vier Cysteinreste durch Alanin ersetzt sind.

10. Hybridprotein nach Anspruch 9, worin die vier Cysteinreste an den Positionen 265, 287, 372 und 379 sind.

11. Hybridprotein nach Anspruch 1 oder Anspruch 8, welches TGF-alphaPE₄₀-Hybridprotein ist, in dem die Cysteinreste an den Positionen 372 und 379 modifiziert sind und in dem die Cysteinreste an den Positionen 265 und 287 unmodifiziert sind.

12. Hybridprotein nach Anspruch 1 oder Anspruch 10, welches ein TGF-alphaPE₄₀-Hybridprotein ist, in dem die Cysteinreste an den Positionen 265, 287, 372 und 379 modifiziert sind.

13. Hybridprotein nach Anspruch 1 oder Anspruch 7, welches ein TGF-alphaPE₄₀-Hybridprotein ist, in dem die Cysteinreste an den Positionen 265 und 287 modifiziert sind und die Cysteinreste an den Positionen 372 und 379 unmodifiziert sind.

14. DNA-Sequenz, die für das Hybridprotein nach irgendeinem der Ansprüche 1 bis 13 kodiert.

15. Plasmid, enthaltend die DNA-Sequenz nach Anspruch 14, welches die Expression der Hybridprotein-DNA-Sequenz in einer rekombinanten Wirtszelle ermöglicht.

16. Verfahren zur Herstellung des Plasmids nach Anspruch 15, um das Hybridprotein herzustellen, welches umfaßt, daß eine geeignete Wirtszelle mit dem Plasmid transformiert wird und die transformierte Wirtszelle unter Bedingungen kultiviert wird, welche die Expression des Hybridproteins ermöglichen.

17. Zusammensetzung, die eine cytotoxisch wirksame Menge des Hybridproteins nach irgendeinem der Ansprüche 1 bis 13 und einen physiologisch annehmbaren Träger enthält.

18. Verwendung des Hybridproteins nach irgendeinem der Ansprüche 1 bis 13 zur Herstellung einer Zusammensetzung, die zur Erzeugung selektiver cytotoxischer Aktivität in einer Säuger-Spezies geeignet ist.

19. Verwendung nach Anspruch 18, worin die Zusammensetzung einen physiologisch annehmbaren Träger enthält.

20. Verwendung des Hybridproteins nach irgendeinem der Ansprüche 1 bis 13 zur Herstellung eines Medikaments zur Verringerung der Proliferation von Keratinozyten.

## Patentansprüche (Patentansprüche für folgende(n) Vertragsstaat(en): ES, GR)

1. Verfahren zur Herstellung eines Hybridproteins, umfassend ein modifiziertes PE₄₀-Protein, worin ein PE₄₀-Protein modifiziert wird durch den Ersatz von mindestens zwei Cysteinresten durch dieselbe Anzahl anderer Aminosäuren, die gleich oder verschieden sein können, mit der Maßgabe, daß sie keine Disulfidbindung bilden, gebunden an eine zielerkennende ("targeting") Proteindomäne, die an einen Wachstumsfaktor-Rezeptor bindet, welches Verfahren umfaßt, daß ein Plasmid, das für das Hybridprotein kodierende DNA enthält, in eine geeignete prokaryotische oder eukaryotische Wirtszelle eingeführt wird und die Wirtszelle unter Bedingungen gezüchtet wird, unter denen das Hybridprotein erzeugt wird.

2. Verfahren nach Anspruch 1, worin mindestens zwei der modifizierten Cysteinreste die Reste 265 und 287 des PE₄₀-Proteins umfassen.

3. Verfahren nach Anspruch 1, worin mindestens zwei der modifizierten Cysteinreste die Reste 372 und 379 des PE₄₀-Proteins umfassen.

4. Verfahren nach irgendeinem der Ansprüche 1 bis 3, worin das modifizierte PE₄₀-Protein modifiziert worden ist, um die Cysteinreste an den Positionen 265, 287, 372 und 379 zu ersetzen.

5. Verfahren nach irgendeinem der Ansprüche 1 bis 4, worin die Cysteinreste gegen eine Aminosäure ausgetauscht sind, die aus der Gruppe aus Alanin, Phenylalanin, Leucin, Isoleucin und Valin ausgewählt ist.

6. Verfahren nach Anspruch 5, worin mindestens zwei der Cysteinreste gegen Alanin ausgetauscht sind.

7. Verfahren nach Anspruch 6, worin die beiden Cysteinreste an den Positionen 265 und 287 des PE₄₀-Proteins sind.

8. Verfahren nach Anspruch 6, worin die beiden Cysteinreste an den Positionen 372 und 379 des PE₄₀-Proteins sind.

9. Verfahren nach irgendeinem der Ansprüche 6 bis 8, worin vier Cysteinreste durch Alanin ersetzt sind.

10. Verfahren nach Anspruch 9, worin die vier Cysteinreste an den Positionen 265, 287, 372 und 379 sind.

11. Verfahren nach Anspruch 1 oder Anspruch 8, worin das modifizierte Protein ein TGF-alphaPE₄₀-Hybridprotein ist, in dem die Cysteinreste an den Positionen 372 und 379 modifiziert sind und die Cysteinreste an den Positionen 265 und 287 unmodifiziert sind.

12. Verfahren nach Anspruch 1 oder Anspruch 10, worin das modifizierte Protein ein TGF-alphaPE₄₀-Hybridprotein ist, in dem die Cysteinreste an den Positionen 265, 287, 372 und 379 modifiziert sind.

13. Verfahren nach Anspruch 1 oder Anspruch 7, worin das modifizierte Protein ein TGF-alphaPE₄₀-Hybridprotein ist, in dem die Cysteinreste an den Positionen 265 und 287 modifiziert sind und die Cysteinreste an den Positionen 372 und 379 unmodifiziert sind.

## Revendications (Revendications pour l'(les) Etat(s) contractant(s) suivant(s): DK, DE, GB, FR, IT, NL, SE, LI, CH, BE, AT, LU)

1. Protéine hybride comprenant une protéine PE₄₀ modifiée, dans laquelle ladite protéine PE₄₀ a été modifiée pour remplacer au moins deux de ses résidus cystéine avec le même nombre d'autres acides aminés qui peuvent être identiques ou différents, à condition qu'ils ne forment pas une liaison disulfure, et dans laquelle ladite protéine PE₄₀ modifiée ou fragment est liée à un domaine de ciblage de protéine qui se lie à un récepteur de facteur de croissance.

2. Protéine hybride selon la revendication 1, dans laquelle au moins deux desdits résidus cystéine qui sont modifiés comprennent les résidus 265 et 287 de la protéine PE₄₀.

3. Protéine hybride selon la revendication 1, dans laquelle au moins deux desdits résidus cystéine qui sont modifiés comprennent les résidus 372 et 379 de la protéine PE₄₀.

4. Protéine hybride selon l'une quelconque des revendications 1 à 3, dans laquelle la protéine PE₄₀ modifiée a été modifiée pour remplacer les résidus cystéine en positions 265, 287, 372 et 379.

5. Protéine hybride selon l'une quelconque des revendications 1 à 4, dans laquelle les résidus cystéine sont changés pour un acide aminé choisi dans le groupe constitué de l'alanine, de la phénylalanine, de la leucine, de l'isoleucine et de la valine.

6. Protéine hybride selon la revendication 5, dans laquelle au moins deux des résidus cystéine sont changés pour l'alanine.

7. Protéine hybride selon la revendication 6, dans laquelle lesdits deux résidus cystéine se trouvent en positions 265 et 287 de la protéine PE₄₀.

8. Protéine hybride selon la revendication 6, dans laquelle lesdits deux résidus cystéine se trouvent en positions 372 et 379 de la protéine PE₄₀.

9. Protéine hybride selon l'une quelconque des revendications 6 à 8, dans laquelle les quatre résidus cystéine sont remplacés par l'alanine.

10. Protéine hybride selon la revendication 9, dans laquelle les quatre résidus cystéine se trouvent en positions 265, 287, 372 et 379.

11. Protéine hybride selon la revendication 1 ou la revendication 8, qui est la protéine hybride TGF-alpha/PE₄₀ dans laquelle les résidus cystéine en positions 372 et 379 sont modifiés et dans laquelle les résidus cystéine en positions 265 et 287 ne sont pas modifiés.

12. Protéine hybride selon la revendication 1 ou la revendication 10, qui est une protéine hybride TGF-alpha/PE₄₀ dans laquelle les résidus cystéine en positions 265, 287, 372 et 379 sont modifiés.

13. Protéine hybride selon la revendication 1 ou la revendication 7, qui est une protéine hybride TGF-alpha/PE₄₀ dans laquelle les résidus cystéine en positions 265 et 287 sont modifiés et les résidus cystéine en positions 372 et 379 ne sont pas modifiés.

14. Séquence d'ADN qui code pour la protéine hybride selon l'une quelconque des revendications 1 à 13.

15. Plasmide contenant la séquence d'ADN selon la revendication 14, qui assure l'expression de la séquence d'ADN de la protéine hybride dans une cellule hôte recombinée.

16. Procédé de production du plasmide selon la revendication 15 pour produire ladite protéine hybride, lequel comprend la transformation d'une cellule hôte adaptée avec ledit plasmide et la culture de ladite cellule hôte transformée dans des conditions qui assurent l'expression de la protéine hybride.

17. Composition contenant une quantité cytotoxique efficace de la protéine hybride selon l'une quelconque des revendications 1 à 13 et un véhicule physiologiquement acceptable.

18. Utilisation de la protéine hybride selon l'une quelconque des revendications 1 à 13 pour la préparation d'une composition utile pour la production d'une activité cytotoxique sélective chez une espèce mammifère.

19. Utilisation selon la revendication 18, dans laquelle la composition contient un véhicule physiologiquement acceptable.

20. Utilisation de la protéine hybride selon l'une quelconque des revendications 1 à 13 pour la préparation d'un médicament destiné à réduire la prolifération des kératinocytes.

## Revendications (Revendications pour l'(les) Etat(s) contractant(s) suivant(s): ES, GR)

1. Procédé de production d'une protéine hybride comprenant une protéine PE₄₀ modifiée, dans lequel une protéine PE₄₀ est modifiée par remplacement d'au moins deux résidus cystéine par le même nombre d'autres acides aminés, qui peuvent être identiques ou différents, à condition qu'ils ne forment pas une liaison disulfure, liée à un domaine de ciblage de protéine qui se lie à un récepteur de facteur de croissance, lequel comprend l'insertion d'un plasmide contenant l'ADN codant pour ladite protéine hybride dans une cellule hôte procaryote ou eucaryote adaptée et la culture de la cellule hôte dans des conditions telles que la protéine hybride est produite.

2. Procédé selon la revendication 1, dans lequel au moins deux desdits résidus cystéine qui sont modifiés comprennent les résidus 265 et 287 de la protéine PE₄₀.

3. Procédé selon la revendication 1, dans lequel au moins deux desdits résidus cystéine qui sont modifiés comprennent les résidus 372 à 379 de la protéine PE₄₀.

4. Procédé selon l'une quelconque des revendications 1 à 3, dans lequel la protéine PE₄₀ modifiée a été modifiée pour remplacer les résidus cystéine en positions 265, 287, 372 et 379.

5. Procédé selon l'une quelconque des revendications 1 à 4, dans lequel les résidus cystéine sont changés pour un acide aminé choisi dans le groupe constitué de l'alanine, de la phénylalanine, de la leucine, de l'isoleucine et de la valine.

6. Procédé selon la revendication 5, dans lequel au moins deux des résidus cystéine sont changés pour l'alanine.

7. Procédé selon la revendication 6, dans lequel lesdits deux résidus cystéine se trouvent en positions 265 et 287 de la protéine PE₄₀.

8. Procédé selon la revendication 6, dans lequel lesdits deux résidus cystéine se trouvent en positions 372 et 379 de la protéine PE₄₀.

9. Procédé selon l'une quelconque des revendications 6 à 8, dans lequel les quatre résidus cystéine sont remplacés par l'alanine.

10. Procédé selon la revendication 9, dans lequel les quatre résidus cystéine se trouvent en positions 265, 287, 372 et 379.

11. Procédé selon la revendication 1 ou la revendication 8, dans lequel la protéine modifiée est une protéine hybride TGF-alpha/PE₄₀ dans laquelle les résidus cystéine en positions 372 et 379 sont modifiés et les résidus cystéine en positions 265 et 287 ne sont pas modifiés.

12. Procédé selon la revendication 1 ou la revendication 10, dans lequel la protéine modifiée est une protéine hybride TGF-alpha/PE₄₀ dans laquelle les résidus cystéine en positions 265, 287, 372 et 379 sont modifiés.

13. Procédé selon la revendication 1 ou la revendication 7, dans lequel la protéine modifiée est une protéine hybride TGF-alpha/PE₄₀ dans laquelle les résidus cystéine en positions 265 et 287 sont modifiés et les résidus cystéine en positions 372 et 379 ne sont pas modifiés.
